# EUROPEAN PATENT APPLICATION

(11) **EP 4 686 492 A1**
(43) Date of publication of application: **04.02.2026**
(21) Application number: 25191680.5
(22) Date of filing: 24.07.2025
(51) Int. Cl.: A61M 37/00

(54) **NEEDLE MODULE AND MOUTHPIECE FOR TATTOO DEVICES**

(30) Priority: 29.07.2024 CN 202411030379
(71) Applicant: Xiao, Long, North York, ON M3A 3H3 (CA)
(72) Inventor: XIAO, Long, North York, ONTARIO, M3A 3H3 (CA); SHENG, Quanming, North York, ONTARIO, M3A 3H3 (CA)
(74) Representative: Gamba, Alessandro

(57) **Abstract**

A mouthpiece (15) of a needle assembly is provided. The needle assembly comprises a needle bundle (200) movably mounted therein. The mouthpiece comprises a tubular wall defining a longitudinal channel (150) allowing for reciprocal movement of the needle bundle; an opening (16) at a terminal end of the longitudinal channel, allowing for ingress and egress of needle tips of the needle bundle from the mouthpiece; and a conjoining and pumping port (180) within 1 mm from the opening, the port configured to allow ink absorbed on the needle tips and the ink on an exterior surface of the tubular wall adjacent to the opening to conjoin into a continuous volume through the port, and to allow the reciprocal movement of the needle bundle to pump the ink in the continuous volume into the longitudinal channel of the mouthpiece from the exterior surface. The ink is water-based and the needle tips comprise a hydrophilic stainless steel.

## Description

### RELATED APPLICATION

This application claims the benefit of and priority from CN patent application no. 202411030379.0, filed July 29, 2024, the entire contents of which is incorporated herein by reference.

### FIELD

The present disclosure relates to devices for tattooing or permanent make-up, particularly to a needle module and a mouthpiece for tattoo devices.

### BACKGROUND

A tattoo device generally includes a tattoo needle for applying ink to the skin, a tattoo machine having a tattoo needle driver, and a handle that connects the tattoo needle to the tattoo machine and can be conveniently held by an operator to manipulate the tattoo needle during use. During operation, the tattoo needle driver drives the tattoo needle to reciprocally extend and retract, thereby repeatedly piercing the skin of the tattooed person. The tattoo needle can be provided in a detachable needle module that can be attached to the handle. The needle module is usually sterilized before use and replaced after each use. It is convenient to use a replaceable and disposable needle module.

The needle module is basically a combination of a tattoo needle bundle (abbreviated as needle bundle) and a needle housing matching the needle bundle and pre-assembled together with the needle bundle. The needle module is also referred to as "tattoo needle module", "cartridge needle", or "tattoo needle module device", and is commonly known as "tattoo needle cartridges" on the market.

The needle module has a needle housing (housing) for mounting the needle bundle therein. The needle housing includes a longitudinal channel and an upper open end, and a lower open end connected to the longitudinal channel. The conventional operating posture of the tattooist is similar to the writing posture. In most cases (generally), the needle tips of the needle bundle are directed downward. The lower part of the needle housing is usually named as the mouthpiece (mouth), and the lower open end is also named as the mouthpiece opening (it may also be referred to as opening of the mouth, needle mouth opening, mouth opening, and it can also be named as opening if there is no ambiguity). The opening allows the needle tips to extend out of the needle housing so that the needle tips can reach the skin. The upper part of the needle housing usually includes a component for mounting the needle module with the handle, and the conventional mounting method is a snap-on (convex and concave snap-on) connection.

For relevant information, refer to the following patents or patent applications: CN patent application CN109069811A (PCT application WO2017178069A1, US patent US11040185B2) with an application date of April 15, 2016; Xiao Long's CN patent CN201595866U published on October 6, 2010; Xiao Long's US patent application US2020/0023175 published on January 23, 2020; Xiao Long's US patent application US2019/0217072 published on July 18, 2019; and CN patent application No. 202210515820.9.

The basic structure of a common tattoo needle bundle comprises a needle group (also referred to as tattoo needles or needle heads) and a needle shaft (also referred to as needle bar). The needle group is usually composed of a plurality of thin needle wires (needles) with sharp tips, and the needles are fixed by soldering. A needle group with a generally cylindrical overall outline formed by a bundled array of multiple thin needle wires is referred to as round needles. Alternatively, multiple thin needle wires can form one or more rows, and the overall outline is generally rectangular, which is referred to as flat needles, such as flat needles (single row) or magnum needles (two rows). Conventional needle groups are divided into two categories, which are round needles and flat needles. The mouthpiece opening has a corresponding shape; for example, the opening corresponding to a round needle bundle is round, and the opening corresponding to a flat needle bundle is rectangular. The size of the opening is usually selected to be able to accommodate to the overall size of the round needle bundle or the flat needle bundle.

Tattooists usually submerge the opening of the needle module and the needle tips extending from the opening into the liquid of the ink container (small ink cups of various sizes) to load or absorb the ink into the mouthpiece longitudinal channel of the needle module. There is a gap between the inner wall of the mouthpiece longitudinal channel and the needle group. Usually, the ink will be sucked into the mouthpiece through capillary action and will wet the inner wall of the channel at the mouthpiece opening. When adding ink to the mouthpiece, the tattooist may also insert the needle tips into the liquid of the ink cup and start the tattoo machine. The tattoo needle bundle that reciprocates up and down will lift the ink into the mouthpiece longitudinal channel. The needle bundle works with a high-frequency linear reciprocating motion. When the needle bundle moves back and forth in the needle housing (usually up and down), the needle tips wetted with the ink rush out of the mouthpiece opening and pierce the skin, then the needle tips retreat to the opening to pick up ink, and then rush out again. This process is repeated to perform the tattooing action. When the ink stored in the mouthpiece longitudinal channel is not enough to fully wet the needle tips, the tattooist will dip and add ink again.

The tattoo process usually involves first transferring the design onto the skin, called stenciling, and then tattooing based on the stencil design. It is well known that in painting, lines are usually used to outline the contours first, and then to create the light and dark transitions of the colors. The same method applies to the tattoo process. The process of using a tattoo needle to outline the contours is called lining. Lining, as the name suggests, is to outline or mark the outline. The tattooist's requirement for lining is that the lines are smooth and natural.

There are two methods for lining which are running the tip method and hanging needle method. The running the tip method is to place the mouthpiece of the needle housing against the skin to trace along the draft (draw the line, run the line). The advantage of this method is that the lines are easily tattooed with ink. Running the tip is sometimes called 'Riding the tube'. This skill automatically controls the needle depth to keep the needle from going too deep into the skin. The disadvantage is that the ink in the mouthpiece can easily spread and puddle onto the skin since the mouthpiece is dragged against the skin, thereby polluting and blocking the transferred draft pattern or ruining the stencil. The hanging needle method is also called the floating needle method, the needle tips extend a long distance from the mouthpiece opening, and the mouthpiece maintains a stable distance from the skin without touching. The advantage of this method is that an operator can clearly see the needles moving on the skin (stencil) during lining, so the operator can accurately draw along the stencil while avoiding spillage and pollution caused by direct contact between the ink in the mouthpiece and the skin. The hanging needle method (floating needle method) allows for more control and precision, especially for fine lines and detailed work. It also helps prevent blowouts (when ink spreads too deep) and patchy lines.

To avoid ink interruption during lining of long lines and frequent ink reloading, tattooists usually involuntarily apply too much ink into the mouthpiece, which often leads to ink spillage. In the hanging needle method, excessive ink usually causes ink drops (ink droplets) to hang on the outer wall of the mouthpiece, thereby causing accidental spillage. In fact, ink drops often hang on the outer wall of the mouthpiece even when there is not enough ink inside the mouthpiece.

From the structural design of the needle module, the reason why the ink drops hang on the outer wall of the mouthpiece is that a certain gap must be retained between the needle group and the inner wall of the mouthpiece longitudinal channel in order to be compatible with needle groups (needle heads) with similar outer diameter profile dimensions and to ensure that needle groups can reciprocate freely and smoothly at high speed. This gap is also the channel for the flow of ink. During the tattoo process, the inner wall of the mouthpiece longitudinal channel of the needle module is not able to hold ink easily. It often happens that the ink inside the mouthpiece flows downward out of the opening due to gravity, adheres to the outer wall of the mouthpiece near the opening, and forms an ink drop. This ink drop gradually becomes larger and drips or spreads onto the skin of the tattooed person, contaminating or covering the tattoo stencil, interfering with the normal work of the tattooist. Especially when the mouthpiece is holding a full amount of ink, or when the gap between the needle group and the inner wall of the mouthpiece longitudinal channel is relatively large, ink spread will occur frequently. In addition, until the ink inside the mouthpiece is exhausted, this ink drop sometime continues to hang on the outer wall near the mouthpiece and cannot be picked up by the needle bundle for effective use.

When a needle module for tattoo devices is working, the following common sense and principles are involved.

Liquids have cohesion and adhesion, both manifestations of molecular attraction. Cohesion allows liquids to resist tensile stress, while adhesion allows liquids to cling to other objects.

The phenomenon that liquids spread along the solid surface and adhere to each other when in contact with solids is also known as liquid infiltration of solids, or wetting. Liquid infiltration occurs when the adhesion is greater than the cohesion force. The better the wettability, the better the liquid can spread and disperse on the solid surface.

At the boundary between liquid and gas, the surface of the liquid or the interface between two immiscible liquids, an extremely small pulling force is generated due to the attraction between molecules. It is assumed that there is a thin film layer on the surface, which bears the tensile force of this surface. This pulling force of the liquid is called surface tension. The surface tension of the liquid causes the liquid surface to minimize its surface area. The drop shape of water or ink is the effect of surface tension.

The capillary phenomenon (also known as capillary action) refers to the phenomenon in which liquid inside thin tubular objects or porous objects flows into thin tubular objects or cracks without applying external force. It is caused by a combination of "adhesion between the liquid and the object" and "surface tension caused by cohesion between liquid molecules". This phenomenon can make liquid overcome gravity and rise. The capillary phenomenon is a liquid interface phenomenon.

The capillary phenomenon occurs when the wettability (attraction to liquid) of the solid surface of the needle channel's inner wall and the wettability of the solid surface of the tattoo needle toward the tattoo ink offset the slight force of gravity. When the needle is dipped into ink, the ink level in the gap rises until it reaches equilibrium with gravity.

The material of tattoo needles is stainless steel, which is a hydrophilic material. It has good adsorption (wettability) for water-based tattoo ink. The outer surface of the tattoo needle group drives the ink attached to its surface to reciprocate during the reciprocating motion. Based on this principle, the tattooist starts the tattoo machine to quickly fill the ink inside the mouthpiece when dipping the needle. The mouthpiece is usually made of plastic, such as transparent PC (polycarbonate) plastic. When the adsorption force of the inner wall of the mouthpiece longitudinal channel on the ink, the adsorption force of the tattoo needle group on the ink, and the self-gravity of the ink in the mouthpiece longitudinal channel reach a balance, the ink can be stably maintained in this space. During the tattoo process, the high-speed reciprocating motion of the tattoo needle drives the ink attached to the surface of the needle group to move, the ink at the needle tips is continuously transferred to the skin surface, and the ink flows downward. Factors such as changes in the angle of the tattoo machine, the vibration of the tattoo machine itself, changes in instantaneous speed, and uneven surface adsorption of the tattoo needle and the position of the plastic mouthpiece cause the flow rate of the ink flowing downward to be unstable. If the ink flowing out of the opening is not transferred to the skin by the needle tips in time, it will gradually accumulate into ink drops and adhere to (attach to) the outer wall of the mouthpiece. If the tattooist dips too much ink when adding ink to the mouthpiece, the ink will move downward due to the influence of gravity. The ink overflowing from the Mouthpiece will accumulate on the outer wall of the mouthpiece to form ink drops under the combined action of the adsorption force on the surface of the outer wall of the mouthpiece (the adsorption force between the contact between the liquid and the solid surface) and the cohesion (surface tension) of the ink itself. When too much ink accumulates to form relatively large ink drops, gravity will be greater than the adsorption force, causing the ink drops will fall off. The ink drops may fall, spread and contaminate the stencil. If the ink drops come into contact with the stainless steel tattoo needle extending out of the mouthpiece opening during the falling process, they will be brought on to the tattooing portion on the skin or be brought back into the mouthpiece longitudinal channel by the reciprocating tattoo needle. It is also possible that the adsorption force continues to be greater than gravity, so the ink drops continue to hang (attach) on the outer wall of the mouthpiece. This part of the ink is not effectively used. These ink drops will also interfere with the tattooist's vision. Because the tattooist cannot be sure whether or when the ink drop will fall off, the ink drop will cause the tattooist to be anxious, affecting and interfering with the tattooist's normal working psychological state.

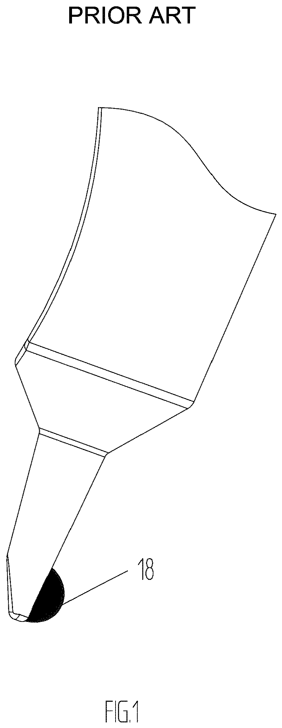

FIG. 1 is a schematic diagram of ink drops appearing on the mouthpiece of a conventional needle module (prior art). As shown in FIG. 1, ink drop 18 appears on the outer wall of the mouthpiece of the needle module. The outer wall of the mouthpiece near the opening is where ink drops often adhere. Since the tattoo posture is similar to the writing posture, the position where ink drops often adhere is the position below the outer wall of the mouthpiece opening.

In the prior art, the mouthpiece longitudinal channel corresponding to the cylindrical round needle group is generally a circular cross-section hole, and the mouthpiece longitudinal channel corresponding to the rectangular cross-section flat needle group is a rectangular opening or slot. Due to factors such as the engineering tolerance in the diameter of the stainless steel needle wire constituting the needle group, the engineering tolerance in the spacing and position of the needle wire array, and the engineering tolerance in the thickness uniformity of the solder layer, the actual cross-sectional shape and size of the needle group will have a relatively large engineering tolerance from the nominal (calibrated, theoretical) shape and size. In order to ensure that the needle group with a large engineering tolerance can also smoothly reciprocate at high speed in the mouthpiece longitudinal channel (usually, 100-150 times per second, and up to 170 times per second at high speed), the inner wall of the needle housing at the mouthpiece longitudinal channel must retain a certain gap with the needle group to avoid jamming. In addition, the size of this gap is usually required to ensure that tattoo inks of different concentrations and viscosities can be dipped, inhaled, stored, and flow smoothly in this channel gap space. Tattoo inks of different brands or colors have different viscosities (fluidity). They also have different adsorption capacities with stainless steel and the plastic surface of the mouthpiece. In addition, tattooists sometimes mix a certain proportion of distilled water to obtain lighter colors. Different inks have different capillary phenomena (adsorption or wetting ability, fluidity) in the channel gap space. The handheld tattoo machine has different tilt angles. The tattoo machine has different vibration frequencies and amplitudes due to different speeds. Various factors will cause the ability to hold ink (adsorption) in the channel gap space to change, which will cause the ink to overflow in the mouthpiece, resulting in the phenomenon of ink drops 18 hanging on the outer wall of the mouthpiece as shown in FIG. 1.

Therefore, there is a need to improve the existing needle module and mouthpiece for tattoo devices.

### SUMMARY

The present disclosure provides a needle module and a mouthpiece for tattoo devices, so as to reduce the phenomenon of ink drops forming on the outer wall of the mouthpiece during operation of the needle module.

In an aspect, the present disclosure provides a needle module for tattoo devices comprising a needle housing and a needle bundle. The needle housing comprises a body module and a mouthpiece disposed at one end of the body module. The body module is provided with a longitudinal channel through which the needle bundle can be movably installed in the longitudinal channel.

One end of the mouthpiece is provided with a mouthpiece opening for the needle tips of the needle bundle to extend out. The interior of the mouthpiece is provided with a mouthpiece longitudinal channel. The mouthpiece longitudinal channel is connected to the longitudinal channel of the body module.

A through hole connected to the mouthpiece longitudinal channel is provided on the outer wall of the mouthpiece near the opening as a conjoining and pumping port, so that the ink can flow back from the conjoining and pumping port into the mouthpiece longitudinal channel. The conjoining and pumping port can be a through hole or a through groove, which is a hole with capillary action. The capillary force can overcome gravity to keep the ink in the hole or groove. The ink kept in the hole or groove can wet the needle tip and continuously supply ink to the needle tip.

The needle housing further comprises a cap portion disposed at the end of the body module opposite the mouthpiece opening. The cap portion comprises a central through hole. The longitudinal channel of the body module is connected to the mouthpiece longitudinal channel and the central through hole of the cap portion. The needle bundle reciprocates longitudinally between a retracted position and an extended position in the longitudinal channel of the body module. The needle tips of the needle bundle extend out of the mouthpiece opening when the needle bundle is in the extended position. The needle module further comprises a driving member. The driving member is configured to generate a longitudinal force to longitudinally drive the needle bundle toward the retracted position. When the needle bundle is in the extended position, the end of its needle shaft is located at the central through hole.

Wherein, near the conjoining and pumping port, the outer contour surface of the needle bundle and the inner wall of the mouthpiece are set to be close together or have a small gap with a capillary effect.

Wherein, the conjoining and pumping port is a long slit shaped opening along the mouthpiece longitudinal direction. Since the liquid can be deformed to adapt to holes of various cross-sections, the specific shape of the slit hole is not limited and may also take the form of an irregular pore.

Wherein, the conjoining and pumping port has a dimension of 0.3 to 1 mm in the transverse direction of the mouthpiece and a length dimension of 1 to 3 mm in the longitudinal direction.

Wherein, the distance between the conjoining and pumping port and the end of the mouthpiece is 0.3 to 1.0 mm. This position is where ink drops are easily formed in conventional mouthpieces.

Wherein, the conjoining and pumping port is a slit groove starting from the end of the mouthpiece and along the longitudinal direction of the mouthpiece. The slit groove can be rectangular but not limited to this shape. It can also be arched, trapezoidal, S-shaped, etc., with rectangular being just one option.

Wherein, the slit groove is a long slit along the longitudinal direction. The longitudinal length of the long slit groove is 1 mm to 3 mm and the shape is rectangular or arched.

Wherein, a plurality of conjoining and pumping ports can be provided.

Wherein, the multiple conjoining and pumping ports are dense small holes or dense small gaps.

Wherein, the multiple conjoining and pumping ports are evenly distributed around the mouthpiece opening.

Wherein, the conjoining and pumping ports or ink reflux grooves can be provided in multiple numbers around the outer wall of the mouthpiece end to allow the ink to reflux into the mouthpiece longitudinal channel at multiple angles or a wide range of angles. For example, 3, 4 or 6 holes can be provided evenly or unevenly around the full 360 degree circumference.

The cross section of the conjoining and pumping port is wider on the outside and narrower on the inside. For the conjoining and pumping port, the hole is smaller at the inner wall of the mouthpiece close to the needle surface, the hole width of the outer wall of the mouthpiece is larger than the hole width at the inner wall. Thus, the hole narrows towards the inside close to the needle surface. Therefore, the capillary force on the inner side of the hole is greater than on the outer side, enabling the capillary force to draw ink inside. This cross section design is also convenient for injection molding.

Wherein, a needle tip observation window is arranged at the end of the mouthpiece. The conjoining and pumping port is arranged on the mouthpiece wall opposite to the needle tip observation window.

Wherein, at least one capillary hole or capillary slit that is connected to the mouthpiece longitudinal channel is provided on the outer wall of the mouthpiece, at a position away from the mouthpiece opening relative to the conjoining and pumping port. The capillary hole or slit is used to store ink and provide improved capillary force (adsorption force) for the ink in the mouthpiece longitudinal channel in order to more effectively overcome the influence of gravity on the ink, slowing down the downward flow speed of the ink.

Wherein, the capillary holes or capillary slits are elongated pores. The extension direction of the capillary holes or capillary slits is generally perpendicular to the longitudinal direction of the mouthpiece. In the use state, the common use posture is that the needle tip is downward, and at this time, the capillary holes or capillary slits are in a horizontal posture. The ink inside is supported by the groove wall against gravity, which provides a better effect.

In another aspect, the present disclosure provides a mouthpiece for tattoo devices. One end of the mouthpiece is provided with a mouthpiece opening allowing the tattoo needle tip to extend out. A mouthpiece longitudinal channel is provided inside the mouthpiece. One or more through holes connected to the mouthpiece longitudinal channel are opened on the outer wall of the mouthpiece near the mouthpiece opening as conjoining and pumping ports, so that ink can flow back from the conjoining and pumping ports into the mouthpiece longitudinal channel.

In some aspects, there is provided a mouthpiece of a tattoo needle assembly, the needle assembly including a needle bundle movably mounted in the needle assembly. The mouthpiece includes a tubular wall. The tubular wall defines a longitudinal channel allowing for reciprocal movement of the needle bundle. The tubular wall also defines an opening at a terminal end of the longitudinal channel, allowing for ingress and egress of needle tips of the needle bundle from the mouthpiece. The tubular wall further defines a conjoining and pumping port within 1 mm from the opening. The port is configured to allow ink absorbed on the needle tips and the ink on an exterior surface of the tubular wall adjacent to the opening to conjoin into a continuous volume through the port, and to allow the reciprocal movement of the needle bundle to pump the ink in the continuous volume into the longitudinal channel of the mouthpiece from the exterior surface. The ink is water-based and the needle tips comprise a hydrophilic stainless steel.

In some embodiments, the port may be sized to generate a capillary force to draw the ink into the port from the exterior surface of the tubular wall.

In some embodiments, the port may be opposite to an observation window of the needle assembly.

In some embodiments, the port may have a transverse dimension of 0.3 to 1 mm and a longitudinal dimension of 1 to 3 mm.

In some embodiments, the port may extend from the opening.

In some embodiments, the port may include a plurality of ports.

In some embodiments, the plurality of ports may include more than two ports disposed circumferentially and equidistantly.

In some embodiments, the port may be tapered inwardly from the exterior surface of the tubular wall towards the longitudinal channel.

In some embodiments, the port may be elongated longitudinally.

In some embodiments, the mouthpiece may further include capillary slits for storing ink, the capillary slits elongated circumferentially.

In other aspects, there is provided a mouthpiece of a tattoo needle assembly, the needle assembly including a needle bundle movably mounted in the needle assembly. The mouthpiece includes a tubular wall. The tubular wall defines a longitudinal channel allowing for reciprocal movement of the needle bundle. The tubular wall also defines an opening at a terminal end of the longitudinal channel, allowing for ingress and egress of needle tips of the needle bundle from the mouthpiece. The tubular wall further defines a conjoining and pumping port within 3 mm from the opening. The port is configured to allow ink absorbed on the needle tips and the ink on an exterior surface of the tubular wall adjacent to the opening to conjoin into a continuous volume through the port, and to allow the reciprocal movement of the needle bundle to pump the ink in the continuous volume into the longitudinal channel of the mouthpiece from the exterior surface. The ink is water-based and the needle tips comprise a hydrophilic stainless steel.

In other aspects, there is provided a tattoo needle assembly including the mouthpiece as described above.

In some embodiments, the tattoo needle assembly may include a body module for mounting the needle bundle and having a proximal end for connecting with a tattoo machine and a distal end, wherein the mouthpiece is at the distal end of the body module.

In some embodiments, the body module may include a biasing element radially biasing a distal portion of the needle bundle towards the port.

In some embodiments, the body module may be integrated with the mouthpiece.

In some embodiments, the mouthpiece may be attachable to the distal end of the body module.

In some embodiments, the needle tips of the needle bundle may have a higher hydrophilicity than the mouthpiece.

In some embodiments, the mouthpiece may include a plastic.

In other aspects, there is provided a method of operating the tattoo needle assembly as described above. The method includes connecting the tattoo needle assembly to a tattoo machine. The method also includes operating the tattoo machine to reciprocate the needle bundle to apply the ink to the skin surface. The method further includes orienting the conjoining and pumping port of the mouthpiece to allow the ink on the exterior surface of the tubular wall of the mouthpiece to enter the port and be pumped into the longitudinal channel by the reciprocal movement of the needle bundle.

In some embodiments, the method may further include contacting ink on the skin surface with the port of the mouthpiece to absorb the ink on the skin surface into the port.

Embodiments of the present disclosure are not limited to applications in a needle module (needle cartridge) in which a tattoo needle bundle and a mouthpiece are pre-assembled. They also have applications to a needle housing or a mouthpiece of a tattoo device in which traditional needle bundles (tattoo needles on a bar) are assembled together with the mouthpieces by a tattooist, achieving the same effect.

In summary, the needle module and mouthpiece for tattoo devices of the present disclosure can reduce or even prevent the phenomenon of ink drops being formed on the outer wall of the mouthpiece during use.

### BRIEF DESCRIPTION OF THE DRAWINGS

The detailed description of the specific embodiments of the present disclosure will refer to the following accompanying drawings, among which:
FIG. 1 is a schematic diagram of ink drops appearing at the mouthpiece of a conventional needle module;
FIG. 2 is a perspective schematic diagram of a tattooing device, according to an embodiment of the present disclosure;
FIGS. 2A, 2B and 2C are respectively a right side view, a front view and a left side view of a needle module for the tattoo device of FIG. 2, according to an embodiment of the present disclosure;
FIG.3 is a perspective schematic diagram of the needle module shown in FIGS. 2A, 2B and 2C;
FIG. 4 is an exploded view of the needle module shown in FIG. 3;
FIG. 5 is a cross-sectional view of the needle module shown in FIG. 2B;
FIG. 6 is a partial cross-sectional view of the needle module of FIG. 3, wherein the cut-away portion shows the conjoining and pumping port near the mouthpiece opening; and
FIGS. 7A-7F are partial schematic diagrams of different needle modules having different embodiments of the conjoining and pumping port.

### DETAILED DESCRIPTION

In overview, the present disclosure provides a mouthpiece for a needle module with a port adjacent to the mouth opening to prevent formation of ink droplets on the mouthpiece exterior surface from ink adsorbed and accumulated on the exterior surface near the mouth opening. The port serves as a connecting port to conjoin the ink adsorbed on the needles inside the needle module and the ink on the exterior surface into a continuous volume of ink through the port. The port also serves as a pumping port for pumping the ink from the exterior surface into the needle module by cohesive forces within the continuous volume of ink when the needles rapidly move back and forth within the needle module.

Conveniently, when the ink adsorbed on the exterior surface is continuously pumped into the needle module during operation, excessive accumulation of ink or formation of ink droplets on the exterior surface near the mouth opening can be prevented. Even if a droplet of ink is somehow formed or deposited on the exterior surface near the port, the droplet of ink can be quickly pumped into the port and into the needle module.

By comparison, when using a conventional needle module with a conventional mouthpiece, ink drops often form on the outer wall of the mouthpiece, and the tattooist needs to carefully operate the tattoo machine to try to prevent the ink drops from falling onto the skin or a stencil on the skin. When the ink inside the conventional mouthpiece is almost exhausted, the ink drops may occasionally flow down to contact the needle tip portion extending out of the mouthpiece and be brought into the mouthpiece by the fast reciprocating needle bundle. However, quite often, the ink drops would remain adhered to the outer wall of the mouthpiece even when there is no ink inside the mouthpiece.

The mouthpiece structure proposed herein significantly increases the chances that any ink drop on the exterior surface will be drawn into the mouthpiece and be utilized, and reduces the chances of, or prevent, formation of ink drops due to accumulation of the ink on the exterior surface in the first place.

As can be understood, to facilitate pumping of ink into the mouthpiece through the port, it would be beneficial that the ink is more adhesive to the needle surfaces than to the wall surfaces of the mouthpiece. For example, when the ink is water-based, it would enhance pumping of the water-based ink when the needles are more hydrophilic than the mouthpiece wall.

Conveniently, the needles may be formed of a stainless steel, which is hydrophilic (having a water contact angle less than 90 degrees), with smooth surfaces, to provide a water contact angle as low as less than 50 or 45 degrees. Typical mouthpiece materials for tattoo needle modules include plastics having higher water contact angles that allow a water-based ink to form surface droplets.

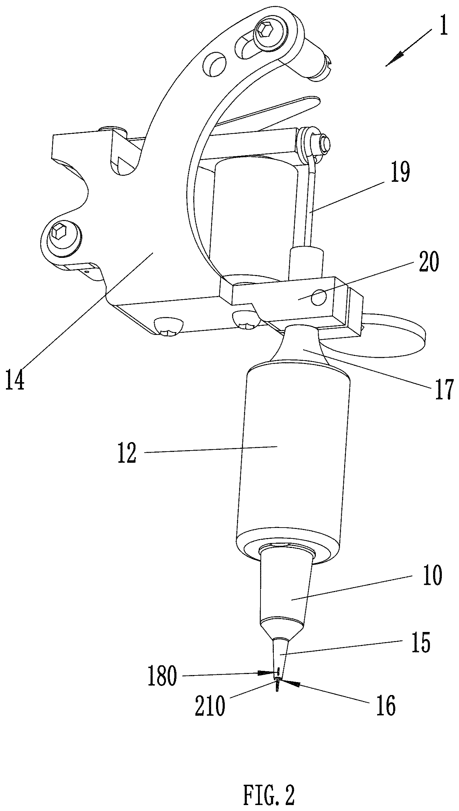

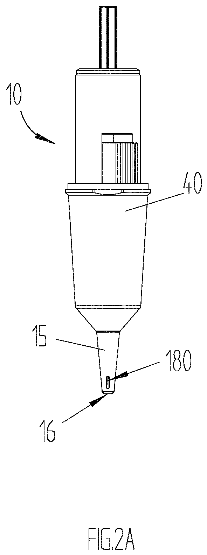

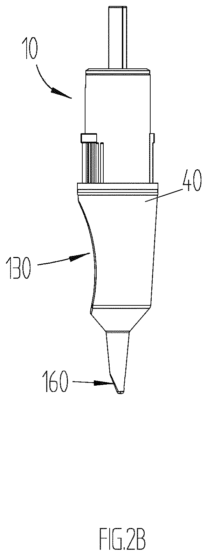

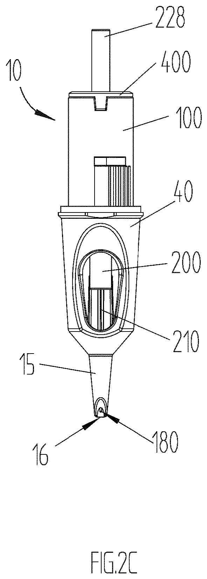

FIG. 2 depicts a tattooing device 1, including a needle module 10, a handle 12 and a base 14. Components of needle module 10 are configured to allow ink to flow from the exterior surface into the needle module 10 (and, in particular, a mouthpiece of the needle module 10) before the ink is accumulated to form any ink drop.

The needle module 10 is configured to be mounted at the bottom end of the handle 12. The needle module 10 includes a mouthpiece 15. Mouthpiece 15 includes opening 16 disposed at the terminal end of mouthpiece 15 and needle module 10. The mouthpiece 15 also includes a conjoining and pumping port 180 close to the opening 16, such as within 3 mm or within 1 mm of opening 16. A needle or one or more needles (needle group 210) in a needle bundle (not depicted) are housed in the needle module 10. The needle bundle and/or needle module 10 may be disposed after each use. The needle bundle is retractable, and the sharp tips of the needle bundle can be hidden in the needle module 10 when not in use. The needle bundle is reciprocally movable within the needle module 10, and the needle module 10 may include a biasing mechanism (not depicted) that biases the needle bundle upwards.

The mouthpiece 15 includes a tubular wall, which may or may not be cylindrical and/or may or may not have a circular cross-section. As well, the tubular wall may not necessarily have an equal cross-sectional size along its length. The tubular wall of mouthpiece 15 may include an exterior wall and define a longitudinal channel within tubular wall, i.e. within mouthpiece 15. The longitudinal channel may terminate in opening 16. The needle or needle bundle may reciprocal longitudinally within the longitudinal channel of the mouthpiece 15.

The handle 12 also has a generally tubular shape, which may be cylindrical as illustrated in the figures. The tubular shape has an inner opening or channel, but the tubular shape may not necessarily be cylindrical or may not necessarily have a circular cross-section. As well, the tubular shape may not necessarily have an equal cross-sectional size along its length. The tubular shape may include an outer profile that is a ball-shape and an inner channel. In further embodiments, the tubular shape may include a vase shape or a bell shape.

A connector 17 is provided and configured to detachably connect the handle 12 to the base 14. The top end of the handle 12 is configured for detachable coupling with the connector 17, such as by a threaded coupling mechanism. The top end of connector 17 is configured to engage the base 14. For clarity, it is noted that for the purpose of this disclosure, the connector 17 is considered a separate part, not necessarily a component of the handle 12.

The base 14 includes a needle actuator 19 having a drive shaft and a clamp 20. As depicted, the top end of connector 17 has a cylindrical shape and can be clamped by clamp 20. The connector 17 has a through opening (not depicted) that allows the drive shaft to pass through.

The clamp 20 may be an adjustable clamp so that the clamping force or pressure can be adjusted. The relative axial position of the connector 17, and hence the handle 12, may be adjusted when the clamp is slightly loosened, and can be secured by tightening the clamp 20.

The bottom end of handle 12 is configured to receive and removably engage the needle module 10.

When the needle module 10, handle 12 and the base 14 are assembled for operation, the needle can be actuated by the actuator 19. In particular, when the drive shaft extends downwards as depicted in the figures, the needle is pushed downwards. When the drive shaft retracts upwards, the needle also retracts upwards due to the internal biasing mechanism. As a result, the actuator 19 and the internal biasing mechanism can drive the needle bundle to move reciprocally up and down for puncturing the skin of a subject.

An ink or other coloured fluid may be applied to the punctured skin using the needle bundle, as can be appreciated by those skilled in the art. The handle 12 is sized and shaped to be conveniently held in a hand of an operator (e.g. tattooist) to apply the ink to the skin.

The construction and operation of the base 14, the handle 12, and the driving mechanism 18 are known in the art and can be similar to, for example, the corresponding parts described in US10806915, US 20160184572, US 20090183602, EP2896427, the entire contents of each incorporated herein by reference. As such, these parts and their operation are not described in detail herein. It is generally noted that common tattoo machines include coil tattoo machines and rotatory tattoo machines. A coil tattoo machine uses electromagnetic coils to move the drive shaft (or an armature bar) up and down. A rotatory tattoo machine is powered by a regulated motor. A tattoo machine may be powered by electricity or a pneumatic fluid (such as compressed air).

In some embodiments, a tattoo needle assembly may include the needle module 10. The needle assembly may also include a needle bundle movably mounted in the needle assembly. In some embodiments, the mouthpiece 15 of the needle assembly may include a tubular wall defining a longitudinal channel allowing for reciprocal movement of the needle bundle. The tubular wall may also define an opening 16 at a terminal end of the longitudinal channel. The opening 16 may allow for ingress and egress of needle tips of the needle bundle from the mouthpiece 15. As used herein, the term "needle tip" includes the very tip of a needle (referred to as a "needle point") and a portion of the needle near the needle point. The needle point and the portion near the needle point can together be referred to as the "tip portion" of the needle. That is, a needle tip as used herein refers to the tip portion of the needle.

The tubular wall may also define a conjoining and pumping port 180, which is within 1 mm or 3 mm from the opening 16 of the mouthpiece 15.

For clarity, it should be noted that, as used herein, the port 180 is "within", e.g., 1 mm or 3 mm, of the opening 16 when the smallest distance between any edge of the port 180 and the terminal end of the mouthpiece 15 is 1 mm or less.

The port 180 may be configured to allow ink absorbed on the needle tips and the ink on an exterior surface of the tubular wall adjacent to the opening 16 to conjoin into a continuous volume through the port 180, and to allow the reciprocal movement of the needle bundle to pump the ink in the continuous volume into the longitudinal channel of the mouthpiece 15 from the exterior surface. The ink may be water-based and the needle tips may include a hydrophilic stainless steel.

Port 180 may be sized to generate a capillary force to draw the ink into the port from the exterior surface of the tubular wall. The port 180 may be opposite to an observation window of the needle assembly.

In some embodiments, the port 180 may have a transverse dimension of 0.3 to 1 mm and a longitudinal dimension of 1 to 3 mm. The thickness of the tubular wall of mouthpiece 15 may be from 0.3 to 1 mm. As such, since the port is defined in the tubular wall, the port 180 may have a depth from 0.3 to 1 mm. The port 180 may also be tapered inwardly from the exterior surface of the tubular wall towards the longitudinal channel.

An edge of the port 180 may be 0 to 1 mm from the terminal end (opening 16) of the mouthpiece 15 and a center of the port 180 may be 1.5 mm (if port 180 is 1 mm long) to 2.5 mm (if port 3 mm long) from the terminal end (opening 16) of the mouthpiece. The face of port 180 may be circular or polygonal.

It will be appreciated that a thinner tubular wall of mouthpiece 15 (e.g. a thickness value closer to 0.3 mm) may improve the operation of needle module 10. For example, a thinner tubular wall of mouthpiece 15 may improve the view of the needles during use for the operator of needle module 10. It may also be advantageous for the tubular wall of mouthpiece 15 at and/or near opening 16 to be thin (e.g. a thickness value closer to 0.3 mm). For example, the thinner the wall at port 180, the easier it may be for ink to be pumped from the exterior surface of the tubular wall of mouthpiece 15 into the longitudinal channel by the reciprocating motion of needle bundle 200. In some embodiments, at port 180, the thickness of the tubular wall of mouthpiece 15 may be from 0.3 to 1 mm.

The port 180 may extend from the opening 16 of mouthpiece 15. The port 180 may also include a plurality of ports. For example, the plurality of ports may include two ports beside one another or more than two ports disposed circumferentially and equidistantly.

The port 180 may be elongated longitudinal. As well, the mouthpiece 15 may further include capillary slits for storing ink, and the capillary slits may be elongated circumferentially.

The tattoo needle assembly may include mouthpiece 15 as described above. The tattoo needle assembly may also include a body module for mounting the needle bundle. The body module may have a proximal end for connecting with a tattoo machine, such as handle 12, base 14 and/or any other components of the tattoo machine. The body module may also have a distal end. Mouthpiece 15 may be disposed at the distal end of the body module. The body module may be integrated with the mouthpiece 15 or the mouthpiece 15 may be attachable to the distal end of the body module.

The body module may include a biasing element radially biasing a distal portion of the needle bundle towards the port.

As well, the needle tips of the needle bundle may have a higher hydrophilicity than the mouthpiece. For example, while the needle tips may include stainless steel (as discussed above), the mouthpiece may include plastic.

In some embodiments, the tattoo needle assembly may be operated using a method including the following steps. The tattoo needle assembly may be connected to a tattoo machine. For example, the tattoo machine may include the handle 12, base 14 and/or any other components of the tattoo machine discussed above. The tattoo machine may be operated to reciprocate the needle bundle to apply the ink to the skin surface. For example, and as already noted above, the needle bundle may reciprocate longitudinally in the longitudinal channel of mouthpiece 15. The conjoining and pumping port 180 of mouthpiece 15 may be oriented to allow the ink on the exterior surface of the tubular wall of the mouthpiece to enter the port and be pumped into the longitudinal channel by the reciprocal movement of the needle bundle.

The method may also include contacting ink on the skin surface with the port 180 of the mouthpiece 15 to absorb the ink on the skin surface into the port 180.

Other method steps may also be possible.

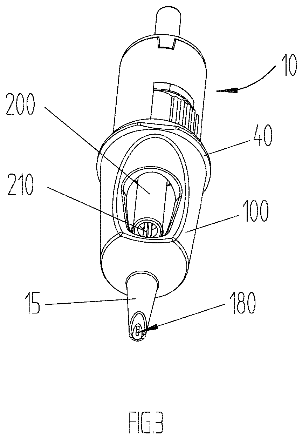

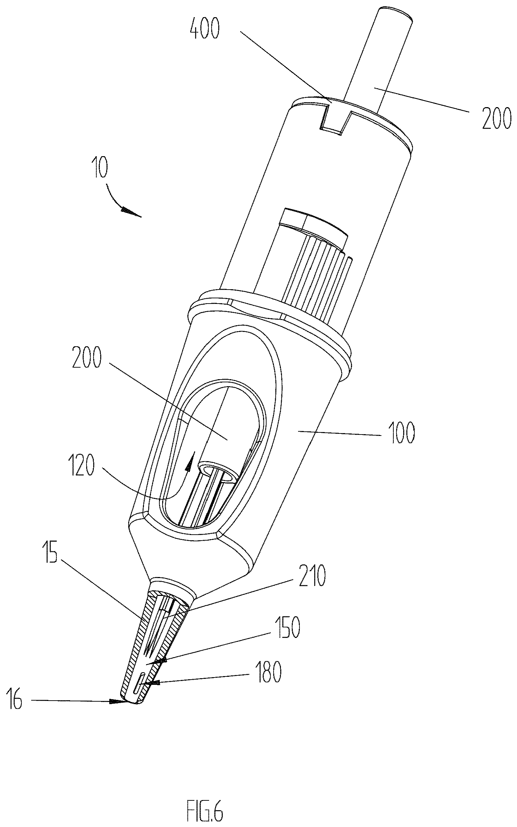

FIGS. 2A-6 illustrate the needle module 10 for tattoo devices, according to an embodiment of the present disclosure. The outer wall of the mouthpiece 15 near the opening 16 is provided with a slit hole leading into the interior of the mouthpiece 15 serving as a conjoining and pumping port 180. During the tattoo process, the tattoo needle reciprocating at high speed can absorb the ink near the slit hole and draw it back (reflux) the mouthpiece longitudinal channel 150 inside the mouthpiece 15.

The tattoo machine and handle for mounting and driving the reciprocating motion of the needle module are not the focus of the present disclosure. They can be implemented by a person skilled in the art according to the prior art or existing structure, as discussed immediately below. Therefore, some details about the tattoo machine and handle are not discussed here.

Referring to FIG. 2A, FIG. 2B, FIG. 2C, FIG. 3, FIG. 4, FIG. 5 and FIG. 6, a needle module 10 of an embodiment of the present disclosure is shown. As is well known in the tattoo industry, the needle module 10 is connected to a tattoo machine (not shown) through a handle (not shown). The tattoo machine has a rod-shaped driving component (not shown) that passes through the center hole of the handle (not shown) and is connected (usually a top-contact connection) to the tail end 228 of the needle shaft of the needle module 10. The driving component of the tattoo machine performs a linear reciprocating motion according to a specific stroke. It is usually only responsible for pushing the needle bundle 200 downward from the retracted position to the extended position. The structure and operation mode of the tattoo machine and the handle can adopt the technology disclosed in US patent applications US11052232, US10806915 or US2019/0217072, the entire contents of each incorporated herein by reference. As a technology or prior art well known to practitioners in the field, the details of the tattoo machine and the handle are therefore not described in detail here.

In this embodiment, the needle module 10 includes a needle housing 40, a needle bundle 200 and a driving member 300.

The needle housing 40 includes a body module 100, a mouthpiece (lower part) 15 provided at the head end of the body module 100, and a cap portion (upper part) 400 which is buckled on (detachably mounted on) the tail end of the body module 100 in a convex-concave bayonet manner. The cap portion 400 has a central through hole 450. The mouthpiece 15 has a mouthpiece longitudinal channel 150. The body module 100 has a longitudinal channel 120. The longitudinal channel 120 of the body module 100 communicates the mouthpiece longitudinal channel 150 of the mouthpiece 15 with the central through hole 450 of the cap portion 400. The mouthpiece longitudinal channel 150 of the mouthpiece 15 has an opening 16, so the opening 16 constitutes the lower opening end of the needle housing 40. The central through hole 450 of the cap portion 400 constitutes the upper opening of the needle housing 40 after the cap portion 400 is buckled on the tail end of the body module 100. In the present embodiment, the mouthpiece 15 and the body module 100 are an integral component (an integrally manufactured part). However, in other embodiments, the mouthpiece 15 and the body module 100 may be two separate parts that are assembled and connected to form a whole.

The body module 100 and the cap portion 400 may be made of plastic, such as polycarbonate.

The needle bundle 200 is installed and guided in the longitudinal channel 120 of the needle housing 40 in a manner that allows the needle bundle 200 to reciprocate up and down during use. The needle bundle 200 has a needle group 210, which is used to extend from the opening 16 to pierce the skin and apply tattoo ink into the skin. The tail end of the needle bundle 200 extends out of the central through hole 450 to contact (impact) with the driving component connected to the tattoo machine and be driven by it.

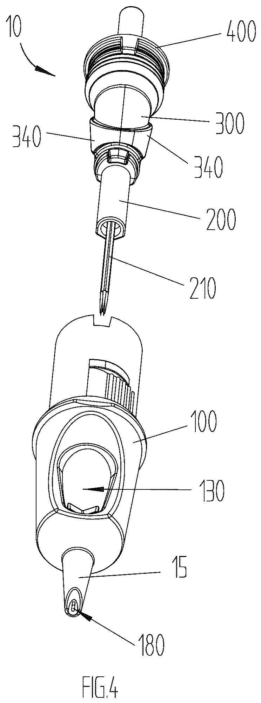

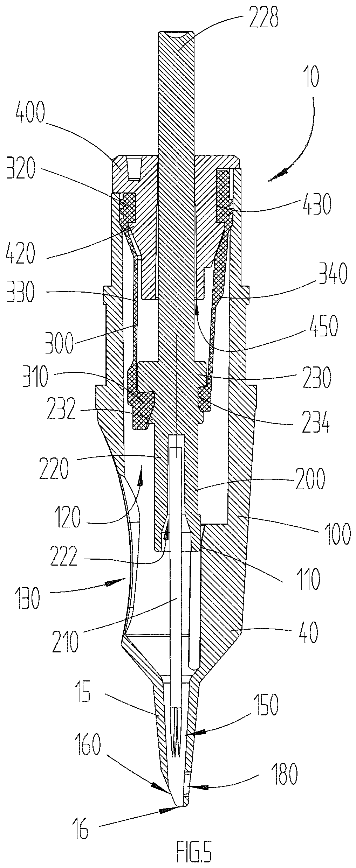

As shown in FIG. 4 and FIG. 5, the needle bundle 200 includes a needle group 210 and a needle shaft 220. The needle group 210 is an array combination of multiple stainless steel thin needles. The cross-sectional profile formed by the array combination of the conventional needle group 210 is generally circular or rectangular. Among the needle modules commonly used in the tattoo field market, a needle group with an array of 1 to 18 stainless steel thin needles forming a circular profile is referred to as "round needle series", such as RL series round liner needles, RS series round shader needles. In the conventional series, the diameter of a single stainless steel needle wire is 0.25 mm, 0.30 mm, or 0.35 mm. A needle group with an array of 4 to 27 stainless steel needles forming a rectangular profile is referred to as "flat needle series", such as F series (single-row linear) flat needles, M1 series (double-row weaved) magnum needles, and RM series (round magnum) needles. The size of the needle can be selected by the operator or user based on the desired effect. Different sizes, different numbers of needles and array arrangement shapes can be used for different purposes. Based on the desired tattooing technique and implementation purpose, a tattooist familiar with this field can select various specifications of the needle group 210.

In the embodiment shown in FIG. 4, FIG. 5 and FIG. 6, the needle group 210 specifically uses RL series round liner needles. It is formed by an array of five needles to form a generally circular cross-sectional profile. The five needles are connected to each other and arranged in a cylindrical array by brazing (soldering). Thus, in this embodiment, the mouthpiece longitudinal channel 150 is also a circular cross-sectional profile. The needle group 210 is inserted into the shaft hole 222 at one end of the needle shaft 220. They form an integrated component by bonding or inlaying to constitute a needle bundle 200. The needle shaft 220 is usually made of plastic, such as polycarbonate. The needle shaft 220 has a neck (clamping portion) 234 and a shoulder 230 for connecting the driving member 300. Referring to FIG. 5, the driving member 300 has a lower end 310 with a central opening. The lower end 310 embraces and clamps the neck 234 of the needle shaft 220. The shoulder 230 of the needle shaft 220 presses against the inner wall of the lower end 310 of the driving member 300, and combined with the limiting function of the collar 232, the lower end 310 of the driving member 300 is connected and fixed at the middle portion or midpoint of the needle bundle 200, that is, at the neck 234.

As shown in FIG.5, the cap portion 400 has a central through hole 450 to allow the tail end 228 of the needle shaft 220 to extend and reciprocate. The tail end 228 of the needle shaft 220 transmits the driving force from the needle driver (not shown) to the needle bundle 200. Generally, the needle driver on the tattoo device pushes the needle shaft 220 to apply a downward force (from top to bottom in FIG.5 and FIG. 2C) to push the needle bundle 200 to move downwardly to the extended position. When the points of the needle group 210 in the needle bundle 200 move to the outside of the needle housing 40 through the mouthpiece longitudinal channel 150 and extends out of the opening 16, the points of the needle group 210 can pierce skin and apply ink to skin to form a tattoo pattern.

Conventional needle drivers do not apply an upward force to the needle bundle 200 to pull the needle bundle 200 back to the retracted position. In order to achieve the reciprocating motion of the needle bundle 200, a driving member 300 is provided inside the needle module 10. The driving member 300, made of an elastic material, is responsible for pulling the needle bundle 200 back from the extended position to the retracted position. With reference to FIG. 2A-5, the driving member 300 is symmetrically provided with obliquely arranged oblique tension ribs 340 on its elastic stretching portion (the side wall of the elastic cylinder) 330. The oblique tension rib 340 can simultaneously generate lateral forces (radial tension, radial bias force components) when stretched. As a result, the driving member 300 can not only bias the needle bundle 200 upwardly (the upper side in FIG. 5) to pull it back to the retracted position, but also drive the needle bundle 200 to bias radially toward the right side, and drive the needle bundle 200 to press against the right side wall of the needle housing 40.

As shown in FIGS. 4 and 5, the driving member 300 includes a lower end 310, an upper end 320, and a tubular (barrel-wall-shaped) elastic stretching portion 330 connecting the lower end 310 and the upper end 320. The oblique tension rib 340 is arranged on the outer surface of the elastic stretching portion 330. As described above, the lower end 310 is connected to the needle bundle 200. The shape and size of the upper end 320 are closely matched (fitted) with the inner wall of the body module 100, and are squeezed into the space formed by the collar ridge 420 of the cap portion 400, the annular groove 430, and the inner wall of the body module 100, so that the upper end 320 is fixedly connected to the needle housing 40.

The driving member capable of generating lateral force may also be in other forms, such as an elastic band, an elastic ring (for example, Xiao Long's Chinese patent CN201595866U published on October 6, 2010) or an elastic cylinder (for example, Xiao Long's U.S. patent application US2020/0023175 published on January 23, 2020 and Xiao Long's U.S. patent application US2019/0217072 published on July 18, 2019), the entire contents of each incorporated herein by reference.

The driving member 300 can be formed of any suitable material with suitable strength and elasticity. Furthermore, the driving member 300 can be replaced by other driving structures, such as O-rings and hook structures. Other driving structures known to those skilled in the art can also be used as long as they provide the required driving force to pull back the needle bundle 200 and drive the needle bundle 200 toward the guide surface. The driving member 300 conveniently provides two driving forces through a simple structure. The optional driving member can include a suitable elastic material, such as silicone, rubber, latex or similar materials.

Typically, the driving member 300 is configured to pull the needle bundle 200 upwardly after the needle bundle 200 is pushed downwardly against the tail end 228 of the needle shaft 220 by the driving component (not shown) of the tattoo machine in each movement cycle. The driving member 300 is not only configured and used to promote the reciprocating motion of the needle bundle 200. Because the oblique tension rib 340 can generate lateral force when stretched, and the body module 100 of the needle housing 40 is provided with a lower guide support member 110 (as shown in FIG. 5), the driving member 300 can also drive (presses, abuts) the needle bundle 200 toward the lower guide support member 110 located in a side direction (right side in FIG. 5) of the needle housing 40, thereby limiting the radial swing of the needle bundle 200 and promoting the precise tattooing of the needle group 210. (See Chinese patent applications 202210515820.9 and 202310143462.8, the entire contents of each incorporated herein by reference). In some other embodiments, the lower guide support member 110 may not be specially provided, but the inner side wall of the mouthpiece longitudinal channel may be directly used as the guide surface. So, the outer contour surface of the needle group 210 can be driven to abut against the inner side wall of the mouthpiece 15 near the conjoining and pumping port 180.

In an embodiment of the present disclosure, as shown in FIG. 2A-6, an elongated slit hole leading to the inside of the mouthpiece longitudinal channel 150 is opened on the outer wall of the mouthpiece 15 near the opening 16 as a conjoining and pumping port 180. The ink overflowing from the opening 16 and hanging on the outer wall of the mouthpiece 15 is connected to the ink inside the mouthpiece longitudinal channel 150 through the conjoining and pumping port 180. The radial force generated by the oblique tension rib 340 when stretched can drive the outer contour surface of the needle group 210 to move towards the inner wall of the conjoining and pumping port 180. The gap between the outer contour surface of the needle group 210 and the inner wall at the conjoining and pumping port 180 can be configured as a zero gap or a small gap with capillary action. The high-speed reciprocating motion of the tattoo needle drives the ink applied to the needle surface to move at a high speed, this movement draws the ink in the conjoining and pumping port 180 together with the ink hanging on the outer wall of the mouthpiece 15 back into the mouthpiece longitudinal channel 150. As a result, the ink overflowing from the opening 16 and hanging on the outer wall of the mouthpiece 15 cannot grow larger or form ink drops because it is continuously sucked back into to the mouthpiece longitudinal channel 150.

Furthermore, the conjoining and pumping port 180 can be a narrow long slit hole. It is a slit with capillary effect on the ink. The capillary force can overcome gravity to keep the ink in the conjoining and pumping port 180. The ink kept in the slit of the conjoining and pumping port 180 can wet the needle tip and continuously supply ink to the needle tip.

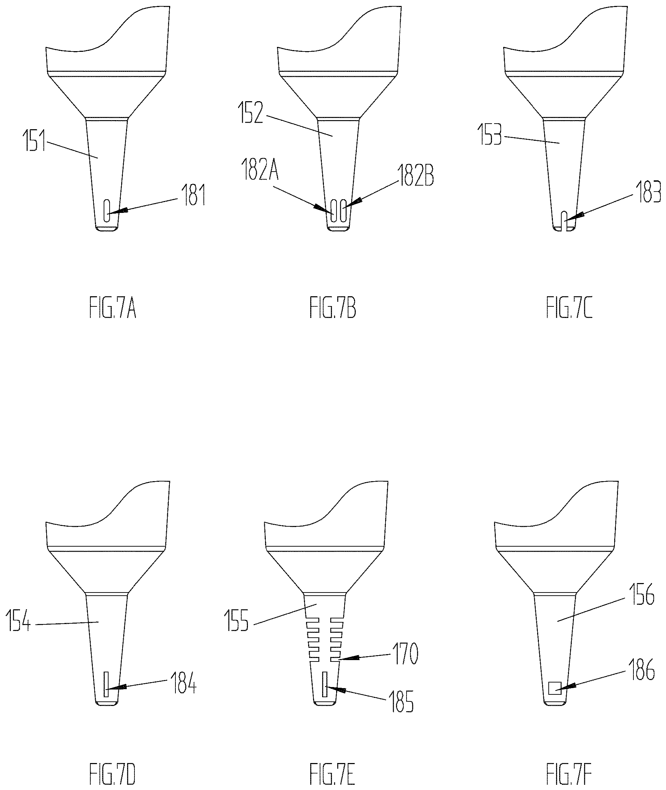

If it is only for eliminating ink drops, the conjoining and pumping port 180 does not necessarily require a strong capillary force, therefore, the conjoining and pumping port 180 can be wider. For example, the conjoining and pumping port 180 has a width dimension of 0.3 to 1mm and a longitudinal length dimension of 1 to 3 mm. If the width dimension is large, a small length dimension can be selected. For example, the conjoining and pumping port 180 can also be a square hole of 1 mm × 1 mm. Because the movement direction of the tattoo needle (needle group 210) is longitudinal, the tattoo needle will have a better effect of sucking back ink when the conjoining and pumping port 180 has a larger longitudinal dimension. The number of conjoining and pumping port could be one. It could be two (as shown in FIG. 7B) or even more for a relatively wide mouthpiece. If the inclination angles and usage habits of the tattoo needle of the tattooist are uncertain, multiple reflux holes or reflux grooves can be opened in a uniform manner within the 360-degree circumference of the mouthpiece, or multiple conjoining and pumping ports (reflux grooves) can be opened in a uniform manner within a relatively large angle range. At the same time, the diameter of the mouthpiece opening and the outer diameter of the tattoo needle are matched to form a small gap (capillary gap), so that the ink on the outer wall of the mouthpiece can be refluxed in a wider range and absorbed by the high-speed moving needle group 210 (tattoo needle) into the mouthpiece longitudinal channel 150 through the conjoining and pumping port. The multiple conjoining and pumping ports can also be dense small holes or dense small gaps. The ink on the outer wall of the mouthpiece can still be absorbed and sucked back into the mouthpiece longitudinal channel 150 as long as the number of small holes is large enough.

Since the ink drops appear at a position very close to the mouthpiece opening 16, the position of the conjoining and pumping port 180 should also be very close to the mouthpiece opening 16. For example, the distance between the edge of the conjoining and pumping port 180 and the opening 16 is 0.3 to 0.8mm. This position is where the ink drops of the conventional mouthpiece are easily formed. Because the tattoo needle with a stainless steel surface exhibits relatively high hydrophilicity, and the stainless steel needle surface is more hydrophilic than the plastic surface of the reflux hole 180, the tattoo needle that reciprocates up and down can bring the ink in the conjoining and pumping port 180 into the mouthpiece longitudinal channel 150. Based on the principle above, when the tattooist adds ink to the mouthpiece 15 (when dipping ink), they usually insert the needle tip into the liquid surface of the ink cup and start the tattoo machine. The tattoo needle that reciprocates up and down will quickly lifting the ink into the mouthpiece longitudinal channel 150, thereby saving time dipping ink. When the tattoo needle is stationary, it takes relatively more time to draw in/absorb the same amount of ink into the mouthpiece longitudinal channel 150.

In addition, it can also eliminate ink drops when the distance between the edge of the conjoining and pumping port and the mouthpiece opening end is 0 mm. Referring to the embodiment shown in FIG. 7C, the conjoining and pumping port 183 is opened as a long slit starting from the end of the mouthpiece opening of the mouthpiece 153 and along the longitudinal direction. In this embodiment, since the ink in the conjoining and pumping port 183 can flow directly downward without obstruction to provide ink for the reciprocating tattoo needle, the ink flow in this embodiment is relatively fast. Experiments have shown that the embodiment shown in FIG. 7C can eliminate ink drops, but the ink flows downward at a high speed, which is suitable for tattooing (lining) dark lines. With the elimination of ink drops as the main purpose, the longitudinal length of the long slit groove of this embodiment should be within the range of 1 to 3mm. If the length is too long, the slit will expand to the channel section causing the ink flow to be too quick. The main function of this slit will transition into an ink delivery groove, which will cause the ink in the longitudinal channel to flow too quickly, thereby making it easy for excessive ink to flow onto the skin. It should be further explained that the function of the conjoining and pumping port 183 is to absorb the ink on the outer wall side of the hole into the mouthpiece longitudinal channel. It can also be used to absorb the ink puddles that have spread onto the skin (the surface tension of the ink forms ink puddles). If the tattooist uses the running the tip (buried needle) method of tattooing (the method of placing the mouthpiece of the needle housing against the skin along the lines of the stencil), one or more conjoining and pumping ports 183 can be opened at the edge of the mouthpiece opening. The function of the conjoining and pumping port 183 can also slow down (reduce) the phenomenon of ink puddles on the skin.

Referring to FIGS. 2A-6, the cross-section of the hole of the conjoining and pumping port 180 is wider on the outside and narrower on the inside, that is, the hole width on the inner wall of the mouthpiece 15 close to the needle surface is small, while the hole width on the outer wall of the mouthpiece 15 is larger than the hole width on the inner wall, and the cross-section of the conjoining and pumping port 180 is a trapezoid. Therefore, the capillary force on the inner side of the hole is greater than that on the outer side. The capillary force can cause the ink to flow inward. This cross-section is also convenient for injection molding because it provides a relatively large draft angle.

In this embodiment, referring to FIG. 2B and FIG. 5, a needle tip observation window 160 is provided at the opening 16, which is a common design of the mouthpiece 15 in prior art, especially for the round mouthpieces used most for lining lines. The function of the needle tip observation window 160 is to facilitate the tattooist to directly see the position of the needle tip reciprocating with the naked eye. The tattooist can line (draw lines) more accurately by seeing the reciprocating needle tip. During use, this needle tip observation window 160 is usually facing the tattooist's face or eyes (upper left in FIG. 2B and FIG. 5), while the position where the ink drops are produced at the conventional tattoo mouthpiece is the outer wall position of the mouthpiece 15 opposite the needle tip observation window 160 (see FIG. 1). Therefore, in this embodiment, the inventor chooses to open the conjoining and pumping port 180 on the outer wall opposite to the needle tip observation window 160 of the mouthpiece 15.

For some round mouthpieces without a needle tip observation window at the mouthpiece opening, multiple conjoining and pumping ports can also be set in the circumferential direction. The inner wall of the mouthpiece longitudinal channel and the outer contour of the needle bundle are matched to a small gap with capillary action. In addition, as shown in FIG. 2B, 4 and 5, usually, the needle module 10 is provided with a vent hole 130 on the body module 100. The purpose of the vent hole 130 is to make the air pressure inside the needle housing 40 consistent with the outside, to avoid the ink spraying due to excessive internal air pressure caused by the reciprocating piston motion of the tattoo needle. Tattooists are accustomed to using this vent hole 130 toward the tattooist's face, so it is also possible to choose to open the conjoining and pumping port on the outer wall of the mouthpiece 15 near the mouthpiece opening 16 on the side opposite to the vent hole 130.

Referring to the embodiment shown in FIG. 7E, in addition to the conjoining and pumping port 185, the mouthpiece 155 has at least one (e.g., 4-10) capillary holes or capillary slits (also named as capillary pore slits) 170 on the outer wall of the mouthpiece 155 connected to the mouthpiece longitudinal channel. The capillary holes or capillary slits 170 are used to store ink and provide better capillary force (adsorption force) for the ink in the mouthpiece longitudinal channel to better overcome the influence of gravity on the ink, slow down the speed of the ink flowing downwardly, and further reduce the excessive ink leakage (downward flow during use) at the source, thereby reducing the accumulation of ink at the mouthpiece opening to form ink drops. It should be noted that even if the mouthpiece 155 has capillary holes or capillary slits 170, when the capillary holes or capillary slits 170 are often full of ink, the conjoining and pumping port 185 is still required to prevent the formation of ink drops. Due to the influence of multiple factors such as different ink colors, different formula ingredients, different concentrations and viscosities, and different hydrophilic properties of the mouthpiece material, it is difficult to give a specific value for the size of the capillary gap. The limit can be determined by the gap in which the ink can overcome gravity and not flow downward. This gap is usually selected to be less than 0.5mm.

Referring to the embodiment shown in FIG. 7E, the capillary holes or capillary slits 170 are elongated pores. Their extension direction is (generally) perpendicular to the longitudinal direction. In the use state, the commonly used posture is with the needle tip pointing downwardly, and the capillary holes or capillary slits 170 are in a horizontal posture. The ink in the capillary holes or capillary slits 170 is supported by the groove wall to overcome gravity, which has a better effect.

As noted above, capillary slits 170 may be used to store ink. It is possible that, during operation, an operator can tilt the needle module 10, such as substantially horizontally, to allow ink adsorbed and accumulated on the exterior surface around the opening 16, such as an ink droplet, to flow towards the capillary slits 170 and be sucked into the capillary slits 170, so as to remove the excess ink and store the removed ink in the capillary slits 170. It will be appreciated that, since capillary slits 170 are remote from opening 16 (i.e. disposed farther away from opening 16 than, for example, port 180), temporary re-orientation (titling) of needle module 10 away from its normal operation orientation would be required to flow the excess ink towards the capillary slits 170.

Conveniently, when the port 180 (such as port 185) is provided and disposed closer to opening 16 of mouthpiece 15 (e.g. within 1 mm to 3 mm of opening 16), it is not necessary to tilt or reorient the needle module 10 away from its normal operation orientation to remove the excess ink through port 180 (or 185). As already described above, excess ink on the exterior surface of mouthpiece 15 and the ink inside the mouthpiece 15 may form a continuous volume through port 180 (or 185), and the reciprocal movement of needle bundle 200 may have the effect of pumping the ink in the continuous volume into the longitudinal channel of the mouthpiece 15 from the exterior surface.

The above description is based on the perspective of using the technical solution of the present disclosure in the needle module of the tattoo device. The focus is on the improvement of the present disclosure over the existing needle module. In addition, for conventional needle modules, the needle housing generally also includes a cap portion provided at the end of the body module away from the mouthpiece opening. The cap portion has a central through hole. The longitudinal channel of the body module connects the mouthpiece longitudinal channel and the central through hole. When the needle bundle is in the extended position, the end of the needle shaft opposite the needle group is located at the central through hole. The driving member is also configured to generate a longitudinal force, which drives the needle bundle longitudinally toward the retracted position. In addition to the driving member being an elastic material, there is also a needle module in which the driving member is a magnet, which interacts with the magnet or electromagnet on the driving component of the tattoo machine to drive the tattoo needle to reciprocate. The cap portion of the needle module equipped with a magnet is usually not provided with an opening.

Moreover, the technical solution of the present disclosure is not limited to application in a needle module (integrated needle) in which the needle bundle and the needle housing are pre-combined. In traditional tattoo equipment, the needle bundle and the needle housing with the mouthpiece are separate parts, not an integrated assembly. The tattooist purchases them separately and needs to install the needle bundle and the needle housing on the tattoo machine and the tattoo handle respectively before use. This technical solution can also be applied to the needle housing of a traditional tattoo instrument, which has the same effect. Similarly, since the mouthpiece and the body module of the needle housing of the needle module (needle cartridge) can be an integral component (an integrally manufactured part) or two separate parts that are assembled together, the present disclosure is also applicable to the mouthpiece as a separate component.

The needle housing of the tattoo device of the present disclosure comprises a body module and a mouthpiece arranged at one end of the body module. The body module comprises a longitudinal channel. The Mouthpiece comprises an opening and a mouthpiece longitudinal channel that connects with the longitudinal channel of the body module. The opening is used for the tattoo needle tip to extend out. A through hole is provided on the outer wall of the mouthpiece near the opening as a conjoining and pumping port. The conjoining and pumping port is connected to the mouthpiece longitudinal channel so as to allow ink to reflux into the mouthpiece longitudinal channel. For details, please refer to the relevant contents in the aforementioned description of the needle module of the present disclosure.

FIGS. 7A-7F are schematic diagrams of conjoining and pumping ports of various other embodiments of the needle module for tattoo devices of the present disclosure. In various embodiments of the present disclosure, the conjoining and pumping port can be in various shapes as long as it can provide a sufficiently large channel for ink reflux.

Referring to FIG. 7A, in this embodiment, the conjoining and pumping port 181 of the mouthpiece 151 has the same width on the inner wall side and the outer wall side of the hole. This solution facilitates machining of the hole.

Referring to FIG. 7B, in this embodiment, the mouthpiece 152 is provided with two conjoining and pumping ports 182A and 182B, that is, more conjoining and pumping ports can be provided for a wider mouthpiece. Alternatively, multiple conjoining and pumping ports can be provided 360 degrees around the mouthpiece, so that no matter which direction the tattooist tilts the tattoo needle, the conjoining and pumping ports will function.

Referring to FIG. 7C, in this embodiment, the conjoining and pumping port 183 is opened as a long slit groove starting from the end of the mouthpiece opening and along the longitudinal direction. In this scheme, since the ink in the conjoining and pumping port 183 can flow directly downward without obstruction to provide ink for the reciprocating tattoo needle, the ink flow in this scheme is relatively fast. Experiments have shown that the embodiment shown in FIG. 7C can eliminate ink drops, but the ink flow is fast, which is suitable for tattooing (lining) dark lines.

Referring to FIG. 7D, this conjoining and pumping port can be rectangular but not limited to rectangular. In other embodiments, it can also be arched, trapezoidal, S-shaped, etc., with rectangular being just one option.

Referring to FIG. 7E, in this embodiment, in addition to the conjoining and pumping port 185, the mouthpiece 155 is provided with at least 1 (for example, 4-10) capillary holes or capillary slits 170 on the outer wall of the mouthpiece 155 that are connected to the mouthpiece longitudinal channel. The capillary holes or capillary slits 170 are used to store ink and provide better capillary force (adsorption force) for the ink in the mouthpiece longitudinal channel so as to better overcome the influence of gravity on the ink, reduce the downward pressure of the ink and slow down the downward flow speed of the ink, further reduce excessive ink leakage (downward flow during use) at the source, and thereby reduce the accumulation of ink at the mouthpiece opening to form ink drops.

### Examples

Experiments and testing have been conducted using example embodiments of a mouthpiece as described herein to verify the actual effects achievable by providing the conjoining and pumping port. The test results demonstrated that even when a large amount of ink was loaded into the mouthpiece longitudinal channel, only a very small amount of ink was accumulated and attached to the outer wall of the tested mouthpiece, and no ink drops were formed on the outer wall during testing.

The tested needle module was a 1205RL round needle cartridge. The diameter of the mouthpiece opening of the round mouthpiece was 1.07 mm. The outline diameter of the tattoo needle of the 1205RL specification was 0.9 mm. The tattoo machine used for the testing ran at a speed of 5600 rpm (the tattoo frequency was 93 Hz). The reciprocating stroke length of the tattoo needle was 3.5 mm. The ink used in the experiments was the LINING BLACK ink from Eternal Ink^{™}.

The ink was loaded by dipping and immersing the opening 16 of the mouthpiece into the ink liquid surface, and the tattoo machine was turned on to make the tattoo needle move up and down to absorb the ink into the mouthpiece longitudinal channel 150. The ink dipping and absorbing time was more than 3 seconds (it could be judged visually that the gap between the mouthpiece longitudinal channel 150 and the needle group 210 had been filled with ink). Lines (lining, tattoo lines) were drawn on the fake skin of silicone material for tattoo training.

### Example I

In some of the experiments, the conjoining and pumping ports used were as shown in FIGS. 2A-6, with various sizes. The hole length was 2 mm, and the inner wall width was 0.3 mm or 0.4 mm. The outer wall width corresponding to the 0.3 mm inner wall width was 0.4mm, 0.5 mm, 0.6 mm, 0.8 mm, or 1.0 mm, respectively. The outer wall width corresponding to the 0.4 mm inner wall width was 0.6 mm, 0.7 mm, 0.8 mm, or 1.0 mm, respectively. No ink drops were observed during testing with any of these tested port dimensions.

### Example II

Furthermore, tests were conducted using a rectangular conjoining and pumping port as illustrated by port 184 in FIG. 7D. For the mouthpiece used in these embodiments, the conjoining and pumping port was 0.5 mm away from the mouthpiece opening (end face). The hole length was 2 mm, the inner wall width was 0.3 mm, and the corresponding outer wall width was 0.4 mm, 0.5 mm, 0.6 mm, and 0.8 mm, respectively. No ink drops appeared during these experiments.

### Example III

Further testing was conducted using a conjoining and pumping port shaped as illustrated by port 183 shown in FIG. 7C. In this example embodiment, the conjoining and pumping port extended from the end face of the mouthpiece opening of the mouthpiece longitudinally to form a long slit groove with a groove width of 0.4 mm and a groove length of 2 mm. No ink drops appeared during testing with this mouthpiece.

### Example IV

In this example, the conjoining and pumping port used was shaped as illustrated by port 186 shown in FIG. 7F. In this example embodiment, the distance between the conjoining and pumping port and the mouth opening (end face) was 0.8 mm, the port width was 1.0 mm, and the port length was 1.0 mm. No ink drops appeared during these experiments.

### Example V

Further testing was conducted using a modified conventional needle module, where a conventional needle module was cut with a blade to provide an irregular-shaped hole of about 1 mm² in cross-sectional size near the mouthpiece opening as the conjoining and pumping port. No ink drops appeared during testing with this modified needle module.

### Conclusion

In summary, the test results show that the needle module and mouthpiece for tattoo devices as described herein can reduce or even prevent the phenomenon of ink drops being formed on the outer wall of the mouthpiece during use.

As described above, for ordinary technicians in this field, various other corresponding changes and modifications can be made based on the technical solutions and technical concepts of the present disclosure. All these changes and modifications should fall within the protection scope as defined in the appended claims of the present disclosure.

## Claims

1. A mouthpiece of a tattoo needle assembly, the needle assembly comprising a needle bundle movably mounted in the needle assembly, the mouthpiece comprising:
a tubular wall defining:
a longitudinal channel allowing for reciprocal movement of the needle bundle;
an opening at a terminal end of the longitudinal channel, allowing for ingress and egress of needle tips of the needle bundle from the mouthpiece; and
a conjoining and pumping port within 1 mm from the opening, the port configured to allow ink absorbed on the needle tips and the ink on an exterior surface of the tubular wall adjacent to the opening to conjoin into a continuous volume through the port, and to allow the reciprocal movement of the needle bundle to pump the ink in the continuous volume into the longitudinal channel of the mouthpiece from the exterior surface,
wherein the ink is water-based and the needle tips comprise a hydrophilic stainless steel.

2. The mouthpiece of claim 1, wherein the port is sized to generate a capillary force to draw the ink into the port from the exterior surface of the tubular wall.

3. The mouthpiece of claim 2, wherein the port is opposite to an observation window of the needle assembly.

4. The mouthpiece of any one of claims 1 to 3, wherein the port has a transverse dimension of 0.3 to 1 mm and a longitudinal dimension of 1 to 3 mm.

5. The mouthpiece of any one of claims 1-4, wherein the port extends from the opening.

6. The mouthpiece of any one of claims 1-5, wherein the port comprises a plurality of ports.

7. The mouthpiece of any one of claims 1-6, wherein the port is elongated longitudinally.

8. The mouthpiece of any one of claims 1-7, further comprising capillary slits for storing ink, the capillary slits elongated circumferentially.

9. A tattoo needle assembly comprising the mouthpiece of any one of claims 1-8.

10. The tattoo needle assembly of claim 9, comprising a body module for mounting the needle bundle and having a proximal end for connecting with a tattoo machine and a distal end, wherein the mouthpiece is at the distal end of the body module.

11. The tattoo needle assembly of claim 10, wherein the body module comprises a biasing element radially biasing a distal portion of the needle bundle towards the port.

12. The tattoo needle assembly of any one of claims 9-11 wherein the mouthpiece is attachable to the distal end of the body module.

13. The tattoo needle assembly of any one of claims 9-12, wherein the needle tips of the needle bundle have a higher hydrophilicity than the mouthpiece.

14. A method of operating the tattoo needle assembly of any one of claims 9-13, comprising:
connecting the tattoo needle assembly to a tattoo machine;
operating the tattoo machine to reciprocate the needle bundle to apply the ink to the skin surface; and
orienting the conjoining and pumping port of the mouthpiece to allow the ink on the exterior surface of the tubular wall of the mouthpiece to enter the port and be pumped into the longitudinal channel by the reciprocal movement of the needle bundle.

15. The method of claim 14, further comprising contacting ink on the skin surface with the port of the mouthpiece to absorb the ink on the skin surface into the port.
